Europäisches Patentamt

(19) European Patent Office

Office européen des brevets

(11) Publication number: **0 025 696**
**B1**

(12) **EUROPEAN PATENT SPECIFICATION**

(45) Date of publication of patent specification: **04.04.84**

(21) Application number: **80303197.0**

(22) Date of filing: **11.09.80**

(51) Int. Cl.³: **A 61 K 9/00,**
**A 61 K 31/415**

(54) Controlled release parasiticidal formulations.

(30) Priority: **12.09.79 US 74681**

(43) Date of publication of application:
**25.03.81 Bulletin 81/12**

(45) Publication of the grant of the patent:
**04.04.84 Bulletin 84/14**

(84) Designated Contracting States:
**DE GB LU NL SE**

(56) References cited:
**WO - A - 80/00659**
**DE - A - 2 606 531**
**FR - A - 2 126 270**
**US - A - 3 887 699**
**US - A - 4 145 433**
**US - A - 4 159 322**

**CHEMICAL ABSTRACTS, vol. 90, nr. 15, April 9,
1979, page 71, abstract 115282u, Columbus,
Ohio, US, R.K. PRICHARD et al.: "Prolonged
administration: a new concept for increasing the
spectrum and effectiveness of anthelmintics"**

(73) Proprietor: **ELI LILLY AND COMPANY**
**307, East McCarty Street**
**Indianapolis Indiana 46285 (US)**

(72) Inventor: **Ludwig, Nelson Henry**
**R.R.8, Box 229**
**Greenfield Indiana (US)**
Inventor: **Boisvenue, Rudolf Joseph**
**329, North State Street**
**Greenfield Indiana (US)**

(74) Representative: **Crowther, Terence Roger**
**European Patent Attorney et al,**
**Erl Wood Manor**
**Windlesham Surrey GU20 6PH (GB)**

Courier Press, Leamington Spa, England.

## Controlled release parasiticidal formulations

Parasites constitute a major problem in animal husbandry. Most animals suffer from microscopic endoparasites such as coccidia, worms and the like. Several methods for partial control of such parasites are known. For example, intestinal parasites are typically controlled by periodic de-worming, for instance by tube worming in the case of equines. Many of the available treatments require the use of extremely toxic substances, for example organophosphates, and heavy metal preparations such as arsenic and mercury formulations.

A major problem exists in the treatment of parasites in domestic animals which are allowed to roam freely over a specified area. For example, animals such as cattle and sheep, which are raised for their human food and clothing products, are often permitted to feed and grow on open grassland, particularly during warm weather grass growing seasons. Also, because herds of such animals are often rather large, individual treatment at short intervals is economically prohibitive. Consequently, such animals are not subject to normal methods of controlling parasites.

The idea of prolonged release formulations for the continuous delivery of an active agent to an animal system over a predetermined period of time is known. For example, Siegrist et al. in U.S. Patent No. 3,535,419, disclose sustained release veterinary compositions useful for ruminant fertility control. As pointed out by *Siegrist et al.*, the current state of development of such sustained release formulations suffers in so many respects that widespread use is precluded. In particular, slow release implants suffer from leaving unwanted residues following payout of the active ingredient, as well as failing to afford sufficiently sharp and consistent endpoints to be predictable.

Reuter et al., in U.S. Patent No. 4,011,312, describe what is said to be a prolonged release drug dosage form useful for the treatment of bovine mastitis. Such formulation comprises an antimicrobial agent dispersed in a matrix of a low molecular weight polyester of glycolic and lactic acids. The polyester utilized is required to contain about 60 to 80 mole percent of glycolic acid and 20 to 40 mole percent of lactic acid, with a molecular weight of less than 2000. Such formulations are said to provide effective medication for up to four to six weeks.

Moreover, many prior art compositions suffer from leaving unwanted residues upon dissolution of the copolymer matrix. This is of utmost importance when the compositions are to be used in animals raised for human food production. Moreover, since many chemical compounds which are effective against diseases at certain dose levels can be lethal to the host animal when administered in excessive amounts, it is imperative that a controlled release formulation be one that does not expose the host animal to lethal doses.

The present invention provides a unique formulation of methyl 5(6)-phenylthio-2-benzimidazole carbamate, (hereinafter fenbendazole) combined with a copolymer of lactic acid and glycolic acid such that fenbendazole is controllably released and total biodegradation of the copolymeric matrix is achieved with no unwanted residue remaining in the animal tissue. Also provided is a formulation which is capable of systemically providing uniform protection against endoparasites for a predetermined period of time.

This invention relates to controlled release fenbendazole formulations and to a method for systemically controlling animal parasites. More particularly, the invention provides a controlled release biodegradable formulation useful in the prolonged therapeutic and prophylatic control of parasites in domestic animals, which comprises 20 to 80 weight percent of fenbendazole intimately dispersed throughout a copolymer derived from 60 to 95 weight percent of lactic acid and 40 to 5 weight percent of glycolic acid, said copolymer having an inherent viscosity of 0.08 to 0.30 when measured in chloroform, and having a weight average molecular weight of 6000 to 35000, and being substantially free of polymerization catalyst. The copolymer matrix is prepared by a novel process which permits the substantially complete removal of toxic catalysts or residues, thus rendering the matrix totally biodegradable into substances common to animal systems. The copolymer itself is the subject of EP—A—80303198.8 (Publication No. 0026599).

A preferred copolymeric matric is one derived from 60 to 90 weight percent of lactic acid and 40 to 10 weight percent of glycolic acid with an inherent viscosity of 0.10 to 0.25.

A more preferred copolymeric matrix utilized in the formulations of this invention is one derived from 70 to 80 weight percent of lactic acid and 30 to 20 weight percent of glycolic acid, with an inherent viscosity of 0.13 to 0.23 and a weight average molecular weight of 15000 to 30000.

Fenbendazole (methyl 5(6)-phenylthio-2-benzimidazole carbamate) is an anthelmintic agent described in Belgian 793,358. This compound is the one formulated for the controlled release according to this invention.

A further embodiment of this invention is the formulation for use in a method of controlled prolonged treatment of domestic animals suffering from endoparasitic infestation and in need of treatment or suspected of being susceptible to endoparasitic infestation which method comprises administering an effective dose of a controlled release endoparasitic formulation which is comprised of 20 to 80 weight percent of fenbendazole intimately dispersed throughout 80 to 20 weight percent of a copolymeric matrix derived from the polymerization of 60 to 95 weight percent of lactic acid and 40 to 5 weight percent of glycolic acid, said copolymer having an inherent viscosity of 0.08 to 0.03 when measured in chloroform, and a weight average molecular weight of 6000 to 35000, and being substantially free of polymerization catalyst. A preferred formulation for use in this method comprises 30 to 70 weight percent of fenbendazole intimately dispersed throughout a copolymer which is derived from 70 to 80 weight percent of lactic acid and 30 to 20 weight percent of glycolic acid, said copolymer having a viscosity of 0.13 to 0.23 and a weight average molecular weight of 15000 to 30000.

This invention provides a controlled release formulation which can be administered therapeutically or prophylactically to an animal suffering from endoparasitic infestation or being prone to development of endoparasitic infestation. Such formulation provides effective treatment and control of endoparasitic infestations for a prolonged period of time following a single administration. Periodic administration of the formulation thus provides indefinite protection to animals. Typically, a single administration of a controlled release formulation of this invention provides effective control of parasitic infestation for a period of about ten to about sixty days.

The formulations provided by this invention require a copolymeric material which is uniquely and ideally suited to the controlled release of an effective amount of a pharmaceutical agent to an animal such that the animal can be effectively treated with a minimum of administrations. Such copolymeric material is prepared by a process which permits the substantially complete removal of polymerization catalyst, thereby permitting the total degradation of the copolymeric matrix in a biological system without the concomitant accumulation of toxic residues in animal tissues. This aspect of the invention is of particular significance in the treatment of animals utilized in the production of meat and other animal products intended for human consumption.

The copolymers required for the formulations of this invention are prepared by condensation of lactic acid and glycolic acid in the presence of a readily removable polymerization catalyst. Such catalysts include strong acid ion-exchange resins in the form of beads or similarly hard structures which are easily removed by filtration or similar techniques. Particularly preferred polymerization catalysts include commercially available strong acid ion-exchange resins such as Amberlite® IR-118(H), Dowex® HCR-W (formerly Dowex® 50W), Duolite® C-20, Amberlyst® 15, Dowex® MSC-1, Duolite® C-25D, Duolite® ES-26 and related strong acid ion-exchange resins. The catalyst is added to a mixture of 60 to 95 weight percent of lactic acid and 40 to 5 weight percent of glycolic acid. The amount of catalyst utilized is not critical to the polymerization, but typically is from 0.01 to 20.0 parts by weight relative to the total weight of combined lactic acid and glycolic acid. The polymerization generally is carried out in the absence of solvents; however, organic solvents such as dimethylsulfoxide or N,N-dimethylformamide can be utilized if desired. The polymerization reaction routinely is carried out in a reaction system equipped with a condensing system, thereby permitting the collection and removal of water that is formed, as well as facilitating the removal of any lactide and glycolide by-products that are formed. The polymerization reaction generally is conducted at an elevated temperature of 100 to 250°C., and at such temperature is usually substantially complete within 3 to 172 hours, normally 48 to 96 hours. Ideally, the reaction can be carried out under a reduced pressure, thereby further faciliating removal of water and by-products.

The copolymer thus formed is readily recovered by simply filtering the molten reaction mixture, for example through a wire screen, to remove substantially all of the strong acid ion-exchange polymerization catalyst. Alternatively, the reaction mixture can be cooled to room temperature and then dissolved in a suitable organic solvent such as dichloromethane or acetone and then filtered by normal means so as to remove the solvent-insoluble strong acid ion-exchange resin. The copolymer then is isolated by removal of the solvent from the filtrate, for instance by evaporation under reduced pressure. Further purification of the copolymer can be accomplished if desired by re-dissolving it in a suitable organic solvent and further filtration, including the use of standard filter aids if desired.

The copolymer thus formed is required in the formulations provided by this invention. Such copolymers, while not amenable to exact structure elucidation, are characterized as having a weight average molecular weight of 6000 to 35000, and ideally 15000 to 30000. The copolymers are unique in that they are classified as high molecular weight substances having an inherent viscosity of 0.08 to 0.30 when measured by standard techniques utilizing an Ubbelohde viscometer in which chloroform has an efflux time of about 51 seconds at 25°C. The inherent viscosity of the copolymers is determined by the following equations:

$$\eta r = t/t_o \qquad \eta inh = \frac{\ln \eta r}{C}$$

wherein

$\eta r$ is relative viscosity;

$t_o$ is efflux time of solvent;

$t$ is efflux time of the solution;

$\eta inh$ is inherent viscosity;

$C$ is concentration in grams per 100 ml. of solvent; and

ln is logarithm.

The copolymers utilized in the formulations of this invention are additionally unique in that they are capable of providing a controlled release of pharmaceutical agents heretofore unavailable in animal tissues.

The formulations comprehended by this invention comprise an effective amount of fenbendazole uniformly admixed and dispersed throughout the copolymeric matrix hereinabove described. The formulations contain 20 to 80 weight percent of fenbendazole, ideally 30 to 70 weight percent.

The formulations provided by this invention can be prepared in any of a number of ways including dry mixing, spray drying, melt extrusion and the like. A preferred method of preparation comprises dissolving a suitable amount of the aforementioned copolymer in a solubilizing organic solvent that is readily removed by evaporation, and then adding the desired amount of fenbendazole, followed by uniform mixing and subsequent removal of the organic solvent. For example, about 50 grams of a copolymer derived from about 80 weight percent of lactic acid and about 20 weight percent of glycolic acid, having an inherent viscosity of about 0.18 and a weight average molecular weight of about 25,000, can be dissolved in 200 to 400 ml. of a suitable organic solvent such as dichloromethane, acetone, dimethyl ether, tetrahydrofuran, chloroform, or the like. Fenbendazole, in the amount of about 50 g., is then added to the dissolved copolymer. The solution thus formed is stirred for uniform mixing and then the solvent is removed by evaporation, thus providing a uniformly mixed formulation of copolymer and fenbendazole in a solid mass. The solid so formed can be ground to uniformity and encapsulated for convenient oral administration to an animal. For instance, the formulation can be administered orally to a range fed calf for effective control of parasites over a prolonged period of time. Such treatment provides uniformly controlled release of fenbendazole to the animal, such that the effective dose is safe for the animal. Said effective dose typically amounts to less than about 500 mg. per animal each day. The novel formulation affords treatment to the animal for as long as 10 to 60 days, typically about 30 days.

The formulations of the invention can alternately be prepared by first dissolving the copolymer and fenbendazole in a suitable organic solvent, followed by removal of the solvent by evaporation, and then the copolymer-fenbendazole formulation can be melted, for example by heating to about 130°C., and the melt can be extruded into rods having a diameter from 1.0 to 10.0 millimeters. The extruded rods can be cut to desired lengths so as to provide the desired specific amount of fenbendazole. For example, a formulation which includes about 50 grams of fenbendazole and about 50 grams of a copolymer derived from 70 to 80 weight percent of lactic acid and 30 to 20 weight percent glycolic acid, said copolymer having an inherent viscosity of 0.13 to 0.23, can be melt extruded into rods having a diameter of about 4.0 mm. Such rods of formulated fenbendazole are, when cooled to room temperature, quite hard and brittle, thereby easily cut, and if desired, ground to uniformity. Such rods can be cut into desired lengths so as to obtain the desired dose of fenbendazole. For example, an extruded rod of 20 to 30 (510—760 mm) can be cut and ground into small particles and passed through an appropriate wire sieve, for example from 60 to 160 mesh (about 250—95 microns), so as to obtain formulated copolymer fenbendazole that is easily encapsulated. Alternatively, the rods thus formed can be cut into sections of 2 to 6 inches (50—150 mm) in length and administered to an animal by subcutaneous implantation. Additionally, the ground formulations can be suspended in a suitable vehicle for convenient subcutaneous or intramuscular injection.

The formulations provided by this invention can contain, in addition to the copolymer matrix and fenbendazole, other substances commonly utilized in medicinal formulations. Diluents, carriers, binders, excipients and adjuvants routinely incorporated in such formulations include gum tragacanth, acacia, corn starch, gelatin, alginic acid, magnesium stearate, aluminum monostearate, span 80, tween 80, sorbitan monostearate, hexaglyceryldistearate, glyceryldistarate, sucrose, lactose, methylparaben, propylparaben, bees wax, mannitol, propylene glycol, microcrystalline, cellulose, calcium silicate, silica, polyvinylpyrrolidone, cetostearyl alcohol, cocoa butter, polyoxyethylene sorbitan monolaurate, ethyl lactate, sorbitan trioleate, calcium stearate, talc and others.

It will of course be recognized that the particular dose of formulated fenbendazole will be determined in part by considerations such as the animal to be treated, the particular parasites to be treated or guarded against, whether the treatment required is therapeutic or prophylactic, the particular biological effects manifested by fenbendazole utilized in the formulation, and the extended period of time over which effective treatment and control is desired. In general, however, this invention contemplates that the typical amount of fenbendazole employed in the formulations will be such that the daily payout of fenbendazole is sufficient to provide effective control to the host animal for a sufficiently prolonged period of time following a single administration. Typically, the dose of fenbendazole, as formulated according to this invention, will be such that the daily payout of fenbendazole will amount to 0.2 to 20 parts per million (wt/vol) when analyzed in terms of blood concentration. Such blood level of fenbendazole can be achieved by administering sufficient controlled released formulation so that the fenbendazole administered amounts to 0.1 to 50 mg/kg of animal body weight. For example, an animal weighing about 500 kg can be given sufficient controlled release formulation so that the dose of fenbendazole will be 0.5 to 25 grams, ideally about 10 grams. The formulations thus administered will give uniform dosing to the animal for an extended period of time, such as about 30 days. If fenbendazole was indeed administered in the amount of 10 grams, the host animal would receive about 300 mg each day, for a daily dosage of fenbendazole of about 0.6 mg/kg of animal body weight. Such dosage is ideally suited for the continuous control and treatment of parasitic infestation in ruminant animals such as cattle and sheep. The formulation is readily encapsulated, for example into an open ended steel cylinder, for convenient oral administration to the reticulo-rumen of the ruminant. The packed cylinder is of sufficient density to remain indefinitely in the reticulo-rumen portion of the ruminant's stomach, thus allowing the controlled release of fenbendazole via the reticulo-rumen.

The controlled release formulations provided by this invention are of particular importance in the treatment and control of endoparasites in cattle. The invention provides formulations and a method of treatment which is ideally suited to use in feed lots as well as to use in range fed animals.

As already pointed out, the active ingredient utilized in the controlled release formulations of this invention is a preferred benzimidazole, namely fenbendazole, (i.e. methyl 5(6)phenylthio-2-benzimidazole carbamate) which is currently in use as a drench and is known as Panacur. This form of administration is of course not applicable to prolonged treatment. The use of fenbendazole is greatly enhanced by the controlled release formulations provided by this invention.

The controlled and continuous release of fenbendazole from the formulations provided by this invention has been demonstrated in a number of *in vivo* experiments. One such evaluation involved the controlled release formulations comprised of a copolymer derived from about 80 weight percent of lactic acid and about 20 weight percent of glycolic acid, having a viscosity of about 0.20 and about 50 weight percent fenbendazole uniformly dispersed throughout. (Designated Formulation A in Table I.) A second formulation was evaluated which comprised a copolymer derived from about 80 weight percent of lactic acid and about 20 weight percent of glycolic acid, having a viscosity of about 0.17 and about 50 weight percent fenbendazole. (Designated Formulation B in Table I.) Formulation B was also tested using about 50 weight percent fenbendazole, about 48 weight percent of the copolymer of formulation B, and about 2 weight percent sodium lauryl sulfate. (Designated Formulation C in Table I.) In one such study, mature cattle were equipped with a fistula for ready access to the reticulo-rumen portion of the stomach. Preweighed steel boluses, containing formulations as described above were placed, via the fistula, into the rumen of each of six heifers. The animals were permitted to graze as desired, and were allowed to drink water freely. The formulation filled bolus was removed from the animals, via the fistula, at 13 day intervals over about a one month test period. Each bolus was weighed to determine the amount of active ingredient which had been administered to each animal, and then each bolus was returned to the reticulo-rumen via the fistula. The payout of active ingredient to each of the six test animals is given in Table I.

TABLE I

Payout of Fenbendazole from formulations placed in the rumen of fistulated cattle
Estimated Fenbendazole payout mg/head/day
(weight loss of the bolus divided by 2)

| Payout periods (days) | Formulation A | | Formulation B | | Formulation C | |
|---|---|---|---|---|---|---|
| | Animal #1 | Animal #2 | Animal #3 | Animal #4 | Animal #5 | Animal #6 |
| 0—14 | 110 | 341 | 113 | 103 | 69 | 108 |
| 14—28 | 504 | 786 | 575 | 596 | 421 | 421 |

According to the data presented in Table I, the average daily payout of fenbendazole, from a formulation of this invention containing 50 weight percent of fenbendazole, is about 307 mg/head/day for animal #1, about 564 mg/head/day for animal #2, about 344 mg/head/day for animal #3, about 350 mg/head/day for animal #4, about 245 mg/head/day for animal #5, and about 265 mg/head/day for animal #6. The average daily payout for all six animals is about 349 mg/head/day.

When the above described *in vivo* experiment on fistulated cattle was repeated using a controlled release formulation comprised of a copolymer derived from about 80 weight percent of lactic acid and about 20 weight percent of glycolic acid, having a viscosity of about 0.20 dl/g. and about 25 weight percent of fenbendazole uniformly dispersed throughout, the payout of active ingredient to each of two test animals is given in Table II.

TABLE II

Payout of Fenbendazole from formulations placed in the rumen of fistulated cattle

| Payout periods (days) | Estimated Fenbendazole payout mg/head/day (averaged) | |
|---|---|---|
| | Animal #1 | Animal #2 |
| 0—14 | 41 | 25 |
| 15—28 | 55 | 63 |
| 29—42 | 80 | 84 |
| 43—56 | 91 | 96 |
| 57—70 | 93 | 102 |

Once all of the formulation contained in the steel capsule has been released, the empty capsule is of such weight that it simply remains in the reticulo-rumen. Additional filled capsules can be administered as needed, and all such capsules can be removed at the time of slaughter. Such removed capsules can be cleaned and repacked with the same or different formulation and re-administered to ruminant animals, thereby adding economical benefits to the present invention.

The method provided herein contemplates administering the controlled release formulations by the route generally recognized as effective for fenbendazole. Methods used can include oral and parenteral administration. The formulations can be suspended in a suitable vehicle such as sesame oil for convenient subcutaneous or intramuscular administration, or alternatively can be implanted. The method provides for the control of parasitic infestation in animals for about ten to about sixty days following a single administration, and longer periods of treatment can be achieved by repeated administration as desired.

In an effort to more fully describe the product of this invention, the following detailed examples are provided by way of illustration.

Example 1

To a 3-neck round bottom flask equipped with a condenser and thermometer were added 864.0 g. of lactic acid, 201.0 g. of glycolic acid and 12.0 g. of Dowex HCR-W2-H ion exchange resin. The mixture was stirred and heated to 130°C. for three hours, during which time 400 ml. of water were distilled and collected. After discarding the water thus produced, stirring and heating were continued and the pressure was gradually reduced by vacuum over three hours, after which time the temperature of the reaction mixture had increased to 150°C. at a final pressure of 5 torr (670 Pa). An additional 12.0 g. of Dowex HCR-W2-H catalyst was added to the reaction mixture, and the mixture then was heated to 170°C. at 5.0 torr (670 Pa) for twenty-four hours, and then at 185°C. at 5.0 torr (670 Pa) for an additional 48 hours. The molten reaction mixture next was filtered to remove most of the ion exchange polymerization catalyst, and the filtrate was allowed to cool to room temperature to give 700 g. of 80 percent lactic—20 percent glycolic copolymer. The copolymer was analyzed by proton nuclear magnetic resonance spectrometry and shown to be comprised of 76 percent by weight of lactic units.

The viscosity of the copolymer was determined in a Ubbelohde viscometer in which chloroform had an efflux time of 51 seconds at 25°C. The copolymer was dissolved in chloroform at a concentration of 0.50 g. per 100 ml. of solvent. Inherent viscosity of the copolymer was then determined according to the formulas:

$$\eta rel = \frac{t}{t_o} \qquad \eta inh = \frac{\ln \eta rel}{C}$$

wherein:

$\eta rel$ = relative viscosity
$t_o$ = efflux time of solvent ($CHCl_3$)
$t$ = efflux time of solution
$\eta inh$ = inherent viscosity
$C$ = conc. in grams/100 ml.

The inherent viscosity of the copolymer thus prepared was determined to be 0.19 dl/g.

Example 2

Following the general procedure set forth in Example 1, 432 g. of lactic acid and 101 g. of glycolic acid were condensed in the presence of a total of 12.0 g. of Amberlyst 15 ion exchange polymerization catalyst to afford 350 g. of a copolymer comprised of about 80 percent lactic units and about 20 percent glycolic units. The copolymer had the following inherent viscosity: 0.18 dl/g.

Example 3

Following the general procedure of Example 1, 422.0 g. of lactic acid were condensed with 144.0

6

g. of glycolic acid in the presence of a total of 12.0 g. of Dowex HCR-W2-H ion exchange polymerization catalyst. After removing the catalyst by filtration of the molten reaction mixture, there was provided 350 g. of a copolymer derived from about 75 percent by weight of lactic acid and about 25 percent by weight of glycolic acid. The copolymer exhibited the following inherent viscosity: 0.19 dl/g.

Example 4

Following the general procedure of Example 1, 1080 g. of lactic acid were condensed with 252 g. of glycolic acid in the presence of a total of 30.0 g. of Dowex HCR-W2-H ion exchange polymerization catalyst to give, after removal of the catalyst, 750 g. of a copolymer which was shown by proton NMR to contain about 79 percent of lactic units and about 21 percent of glycolic units. The copolymer exhibited the following inherent viscosity: 0.20 dl/g.

Example 5

Following the procedure of Example 1, 1080 g. of lactic acid were condensed with 120 g. of glycolic acid in the presence of a total of 15.0 g. of Dowex HCR-W2-H ion exchange polymerization catalyst to provide, after work-up, 630 g. of a copolymer derived from about 90 weight percent of lactic acid and about 10 weight percent of glycolic acid. The copolymer had an inherent viscosity of 0.20 dl/g.

Example 6

Following the procedure of Example 1, 710 g. of lactic acid were condensed with 190 g. of glycolic acid in the presence of a total of 12.0 g. of Dowex HCR-W2-H ion exchange polymerization catalyst to provide 500 g. of a copolymer comprised of about 70 percent lactic units and about 30 percent glycolic units. The copolymer had an inherent viscosity of 0.12 after 24 hours at 175°C.

Example 7

The procedure of Example 1 was followed to condense 1080 g. of lactic acid with 120 g. of glycolic acid in the presence of a total of 30.0 g. of Dowex HCR-W2-H ion exchange polymerization catalyst. After workup, there was recovered 750 g. of a copolymer derived of about 89 weight percent of lactic acid and about 11 weight percent of glycolic acid having an inherent viscosity of 0.20 dl/g.

The copolymers provided by this invention additionally have been characterized by gel permeation chromatography (high pressure liquid chromatography) and subsequent determination of molecular weight. Gel permeation chromatography separates sample molecules by differences in effective molecular size in solution. Separation is accomplished as a result of the pore size distribution in the packing material. This analytical technique allows determinations of weight average molecular weight, number average molcular weight, molecular weight distribution, and dispersity for polymeric materials.

Several such experiments have been carried out on the copolymers of this invention. Standard gel permeation chromatographic columns were used, and the support in each case was commercial μStyragel. All samples and standards were dissolved in a solution of 80 parts tetrahydrofuran and 20 parts dichloromethane. The indirect method (i.e. the "Q-Factor Method") of calibrating the gel permeation chromatographic columns was used to obtain molecular weight averages for the copolymers of the invention. Commercial polystyrene, with a Q Factor of 41.3, was used in the calibrations. The following Table presents several determinations of molecular weight by standard gel permeation chromatographic techniques as outlined above. A more detailed discussion of the technique utilized is presented by Slade in *Polymer Molecular Weights,* Marcel Deckker, Inc., 1975.

In the Table, column I presents the relative proportions of lactic units and glycolic units making up the copolymer analyzed. Column II gives the inherent viscosity of each copolymer analyzed. Column III reports the strong acid ion exchange resin utilized to prepare the copolymer being analyzed. Column IV presents the weight average angstrom size as determined from the gel permeation chromatographic retention time for the particular copolymer. Column V presents the weight average molecular weights for the various copolymers prepared by the process of this invention. The weight average molecular weights are determined by multiplying the Q-Factor for polystyrene (41.3) times the weight average angstrom size for the particular copolymer being analyzed. Column VI is the relevant Example number.

As demonstrated in the Table, the preferred copolymers of this invention have a molecular weight from 15,000 to 35,000, and ideally from 15,000 to 30,000.

7

**0 025 696**

Table of weight average molecular weights

| Column I | II | III | IV | V | VI |
|---|---|---|---|---|---|
| 80:20 | 0.19 | Dowex HCR-W2-H | 432.5 | 17,862 | 1 |
| 80:20 | 0.18 | Amberlyst 15 | 412.3 | 17,027 | 2 |
| 75:25 | 0.19 | Dowex HCR-W2-H | 505.9 | 20,894 | 3 |
| 80:20 | 0.20 | Dowex HCR-W2-H | 777.1 | 32,094 | 4 |
| 90:10 | 0.20 | Dowex HCR-W2-H | 749.3 | 30,946 | 5 |
| 70:30 | 0.12 | Dowex HCR-W2-H | 400.5 | 16,541 | 6 |
| 90:10 | 0.20 | Dowex HCR-W2-H | 522.1 | 21,563 | 7 |

Example 8
Formulation for control of endoparasites in ruminants

A uniform dry mixture made up of 200 grams of methyl 5(6)-(phenylthio)benzimidazole carbamate, (i.e. fenbendazole) and 200 grams of a copolymer derived from about 80 weight percent lactic acid and about 20 weight percent of glycolic acid, said copolymer having an inherent viscosity of about 0.18, is heated to about 90°C. The heated mixture is extruded at that temperature through a standard killion extruder, and the extruded product is packed into steel capsules measuring 35 mm×50 mm and weighing about 100 grams when empty. Each capsule will contain about 40 grams of the formulation, thus containing about 20 grams of active ingredient. Such steel capsules containing the controlled release formulation of fenbendazole can be administered to ruminant calves weighing about 500 kg for the prolonged treatment and control of intestinal parasites. The steel capsule thus administered is of sufficient weight that it is retained in the reticulo-rumen portion of the ruminant's stomach. The formulation thus administered is effective for providing controlled and continuous release of active ingredient to such animal for a period of about ten to about sixty days, so that the average daily dose of active ingredient is about 0.5 to about 1.0 mg/kg of animal body weight.

## Claims

1. A controlled release biodegradable formulation useful in the prolonged therapeutic and prophylactic control of parasites in domestic animals which comprises 20 to 80 weight percent of fenbendazole intimately dispersed throughout a copolymer derived from 60 to 95 weight percent of lactic acid and 40 to 5 weight percent of glycolic acid, said copolymer having an inherent viscosity of 0.08 to 0.30 when measured in chloroform, and having a weight average molecular weight of 6000 to 35000, and being substantially free of polymerisation catalyst.

2. The formulation of claim 1 wherein fenbendazole is present in the amount of 30 to 70 weight percent and the copolymer is derived from 60 to 90 weight percent of lactic acid and 40 to 10 weight percent of glycolic acid, with an inherent viscosity of 0.10 to 0.25.

3. The formulation of claim 2 wherein the copolymer is derived from 70 to 80 weight percent of lactic acid and 30 to 20 weight percent of glycolic acid, with an inherent viscosity of 0.13 to 0.23 and a weight average molecular weight of 15000 to 30000.

4. The controlled release formulations of any one of claims 1—3 for use in a method of controlled prolonged treatment of domestic animals suffering from endoparasitic infestation and in need of treatment or suspected of being susceptible to endoparastic infestation which method comprises administering an effective dose of the controlled release endoparasitic formulation.

## Patentansprüche

1. Bioabbaubare Formulierung mit gesteuerter Wirkstoffreigabe zur längerzeitigen therapeutischen und prophylaktischen Bekämpfung von Parasiten bei Haustieren, dadurch gekennzeichnet, daß sie 20 bis 80 Gewichtsprozent Fenbendazol in inniger Verteilung in einem Copolymerisat aus 60 bis 95 Gewichtsprozent Milchsäure und 40 bis 5 Gewichtsprozent Glykolsäure enthält, wobei dieses Copolymerisat eine in Chloroform gemessene inhärente Viskosität von 0,08 bis 0,30 hat und ein gewichtsmittleres Molekulargewicht von 6000 bis 35 000 besitzt und praktisch frei an Polymerisationskatalysator ist.

2. Formulierung nach Anspruch 1, dadurch gekennzeichnet, daß das Fenbendazol in einer Menge von 30 bis 70 Gewichtsprozent vorhanden ist und das Copolymerisat aus 60 bis 90 Gewichtsprozent Milchsäure und 40 bis 10 Gewichtsprozent Glykolsäure besteht und eine inhärente Visckosität von 0,10 bis 0,25 hat.

3. Formulierung nach Anspruch 2, dadurch gekennzeichnet, daß das Copolymerisat aus 70 bis 80 Gewichtsprozent Milchsäure und 30 bis 20 Gewichtsprozent Glycolsäure besteht, eine inhärente Viskosität von 0,13 bis 0,23 hat und eine gewichtsmittleres Molekulargewicht von 15 000 bis 30 000 aufweist.

4. Verwendung der bioabbaubaren Formulierung mit gesteuerter Wirkstoffreigabe gemäß einem

**0 025 696**

der Ansprüche 1 bis 3 bei einem Verfahren zur gesteuerten, längerzeitigen Behandlung von Haustieren, die von Endoparasiten befallen sind und einer solchen Behandlung bedürfen oder bei denen die Gefahr eines Befalls durch Endoparasiten besteht, durch Verabreichung einer wirksamen Dosis einer solchen endoparasitischen Formulierung mit gesteuerter Wirkstofffreigabe an Haustiere.

**Revendications**

1. Formulation biodégradable à libération contrôlée utile dans le contrôle thérapeutique et prophylactique prolongé des parasites chez les animaux domestiques, caractérisée en ce qu'elle comprend 20 à 80% en poids de fenbendazole dispersé intimement dans un copolymère dérivant de 60 à 95% en poids d'acide lactique et de 40 à 5% en poids d'acide glycolique, ce copolymère ayant une viscosité inhérente de 0,08 à 0,30, mesurée dans du chloroforme, tandis qu'il a un poids moléculaire moyen en poids de 6.000 à 35.000 et qu'il est pratiquement exempt de catalyseur de polymérisation.

2. Formulation suivant la revendication 1, caractérisée en ce que le fenbendazole est présent en une quantité de 30 à 70% en poids, tandis que le copolymère dérive de 60 à 90% en poids d'acide lactique et de 40 à 10% en poids d'acide glycolique, ce copolymère ayant une viscosité inhérente de 0,10 à 0,25.

3. Formulation suivant la revendication 2, caractérisée en ce que le copolymère dérive de 70 à 80% en poids d'acide lactique et de 30 à 20% en poids d'acide glycolique, tandis qu'il a une viscosité inhérente de 0,13 à 0,23 et un poids moléculaire moyen en poids de 15.000 à 30.000.

4. Formulation à libération contrôlée suivant l'une quelconque des revendications 1 à 3 en vue de l'utiliser dans un procédé de traitement prolongé et contrôlé d'animaux domestiques souffrant d'une infestation endoparasitaire et nécessitant un traitement ou soupçonnés d'être susceptibles aux infestations endoparasitaires, ce procédé consistant à administrer une dose efficace de la formulation endoparasitaire à libération contrôlée.

9